# EUROPÄISCHE PATENTSCHRIFT

(11) **EP 0 928 597 B1**
(45) Veröffentlichungstag und Bekanntmachung des Hinweises auf die Patenterteilung: **06.03.2002**
(21) Anmeldenummer: 98104877.0
(22) Anmeldetag: 18.03.1998
(51) Int. Cl.: A61B 5/00, A61B 1/04

(54) **Diagnosegerät zur bildgebenden Aufnahme fluoreszierender biologischer Gewebebereiche**
Diagnostic apparatus for imaging fluorescent biological tissues
Appareil de diagnostique pour l' imagerie de tissus biologiques fluorescents

(30) Priorität: 07.01.1998 DE 19800312
(43) Veröffentlichungstag der Anmeldung: 14.07.1999
(73) Patentinhaber: Richard Wolf GmbH, 75438 Knittlingen (DE)
(72) Erfinder: Wagnières, Georges, Dr., 1110 Morges (CH); Zellweger, Matthieu, 1007 Lausanne (CH); Chauvin, Nicolas, Dr., 1018 Lausanne (CH); Lange, Norbert, Dr., 1004 Lausanne (CH); Zanger, Ulf, 76356 Weingarten (DE); Studzinski, André, 1814 La Tour-de-Peilz (CH); van den Bergh, Hubert, Prof. Dr., 1376 Goumoens la Ville (CH)
(74) Vertreter: Wilcken, Thomas, Dipl.-Ing.

(56) Entgegenhaltungen:
- EP-A- 0 512 965
- EP-A- 0 792 618
- EP-A- 0 805 348
- US-A- 5 590 660

## Beschreibung

Die Erfindung betrifft ein Diagnosegerät zur bildgebenden Aufnahme fluoreszierender biologischer Gewebebereiche, insbesondere durch ein Endoskop, mit einer Lichtquelle, die unter Verwendung einer Filteranordnung wenigstens einen zur Fluoreszenzanregung im Gewebe geeigneten Spektralbereich emittiert, mit einer Optik zur Erfassung und Leitung des vom Gewebe reflektierten Fluoreszenzlichts, mit mindestens einer Videokamera, die Bilder von wenigstens zwei verschiedenen Spektralbereichen des vom Gewebe reflektierten und von der Optik geleiteten Fluoreszenzlichts aufnimmt, und mit Bildverarbeitungsmitteln, die durch Verarbeitung der Bildinformation der von der Videokamera aufgenommenen Bilder der verschiedenen Spektralbereiche gemäß einem bestimmten Verarbeitungsalgorithmus in wenigstens zwei getrennten Kanälen jeweils zur Diagnose des untersuchten Gewebebereichs dienende farbseparate Bildsignale erzeugen, wobei die farbseparaten Bildsignale an den Ausgängen der getrennten Kanäle einem RGB-Farbmonitor eingegeben werden, so daß dieser ein gemischtfarbiges Monitorbild zeigt.

Bei einem solchen Diagnosegerät (EP 0 792 618 A1) wird das vom untersuchten Gewebebereich reflektierte Licht mittels eines einen dichroitischen Spiegel aufweisenden Farbteilers in einen roten und grünen Spektralbereich geteilt. Der rote Spektralbereich wird dann getrennt vom grünen Spektralbereich jeweils von einer bildverstärkten CCD-Festkörperkamera aufgenommen. Die analogen Ausgangssignale der Rot- und Grün-Videokameras werden nach Verstärkung und Analog-Digitalumsetzung einem jeweiligen Farbkanal eines einen Mikrocomputer enthaltenden Videoprozessors zugeführt. Die digitalisierten Bildsignale werden vom Videoprozessor hinsichtlich der relativen Verstärkung der digitalen Videosignale modifiziert und daraufhin wieder in analoge Bildsignale zurückgewandelt, die gleichzeitig einem Rot- und einem Grüneingang eines RGB-Farbmonitors zugeführt werden, der ein Falschfarbenbild erzeugt, indem gesundes biologisches Gewebe cyanfarbig und Krebsgewebe rot erscheint.

Weitere ähnlich aufgebaute Diagnosegeräte zur bildgebenden Aufnahme fluoreszierender biologischer Gewebebereiche, insbesondere mit Hilfe eines Endoskops, sind jeweils in EP 0 512 965 A1, US 4 821 117, DE 196 08 027 A1, EP 805348 und in SPIE Band 1203 (1990), Seiten 43-52 beschrieben.

Alle zuvor erwähnten bekannten Diagnosegeräte zur bildgebenden Aufnahme fluoreszierender biologischer Gewebebereiche arbeiten mit zwei bildverstärkten Kameras oder CCD-Festkörper-Pick-Ups. Zum Teil verwenden sie auch einen Laser als Lichtquelle. Aufgrund der doppelten Ausführung der Videokameras wird der Kamerakopf, welcher am Endoskop zu befestigen ist, entsprechend schwer und sperrig und behindert beträchtlich die Handhabung des Endoskops.

Angesichts der Probleme der aus dem oben genannten Stand der Technik bekannten Diagnosegeräte liegt der Erfindung die Aufgabe zugrunde, ein Diagnosegerät so auszubilden, daß es mit bekannten Endoskopen und Zubehör kompatibel ist, einen einfachen Aufbau und geringen Preis hat, sehr kompakt insbesondere hinsichtlich der am Endoskop zu befestigenden Teile ist, daß es die Handhabung des Endoskops möglichst wenig beeinträchtigt und daß auf einen PC als Prozessororgan verzichten werden kann.

Das Diagnosegerät soll den physikalischen Effekt der Autofluoreszenz biologischer Gewebebereiche ausnutzen und eine bildhafte Darstellung der betrachteten Gewebeoberfläche liefern, so daß es möglich ist, tumoröse und prätumoröse Veränderungen von Gewebe in einem möglichst frühen Stadium aufzufinden und ihre Ausdehnung zu bestimmen. Hierzu gehören insbesondere Dysplasien, Karzinoma in situ und kleine papilläre Tumore, welche mit konventionellen Verfahren, beispielsweise endoskopischer Betrachtung unter Weißlicht, nur schwer oder gar nicht erkannt werden können.

Das Diagnosegerät soll auch ohne einen sogenannten Photosensitizer auskommen, so daß dem Patienten kein spezielles Medikament zur Fluoreszenzanregung des Gewebes verabreicht werden muß. Prinzipiell kann das System jedoch auch mit einem geeigneten Photosensitizer arbeiten.

Ein diese obige Aufgabe lösendes geeignetes Diagnosegerät der eingangs erwähnten Art ist erfindungsgemäß dadurch gekennzeichnet, daß die Videokamera eine hochempfindliche Schwarz-Weiß-CCD-Festkörperkamera ist und einen Farbteiler aufweist, der die mindestens zwei Spektralbereiche des empfangenen Lichts in wenigstens zwei räumlich getrennte, dem jeweiligen Spektralbereich zugeordnete parallele Bildbereiche auf dem Schwarz-Weiß-CCD-Festkörper zerlegt, und daß die Bildverarbeitungsmittel die räumlich getrennten Bildbereiche auf dem CCD-Festkörper zeitsequentiell empfangen und in den getrennten Kanälen gegeneinander so verzögern, daß an den Ausgängen der Kanäle die vorher zeitlich getrennten farbseparaten Bildsignale zeitgleich ausgegeben werden.

Somit besteht das Grundprinzip des erfindungsgemäßen Diagnosegeräts in einer spektralen Auswertung der Fluoreszenzinformation zumindest in den Bereichen grün und rot mit einer einzigen hochempfindlichen Schwarz-Weiß-Kamera bzw. einem Schwarz-Weiß-CCD-Festkörperbildaufnehmer statt mit zwei gleichartigen Farbvideokameras. Diese eingesetzte Schwarz-Weiß-Kamera ist erheblich empfindlicher als vergleichbare Farbkameras und damit geeignet, auch lichtschwache Fluoreszenzereignisse ohne Bildverstärker aufzunehmen. Sie kann außerdem mehrere Bilder integrieren und damit die Lichtempfindlichkeit weiter steigern. Mit den erfindungsgemäßen Bildverarbeitungsmitteln läßt sich aus den Informationen der Schwarz-Weiß-Videokamera ein rot-grünes Bild auf einem konventionellen RGB-Videomonitor darstellen. Die jeweilige Farbgebung des Gewebebereichs stellt die Information über den Zustand des Gewebes dar.

Der Farbteiler der Festkörperkamera zerlegt des Fluoreszenzbild des aufgenommenen Gewebebereichs in einen ersten, der Farbe grün zugeordneten Bildbereich und einen davon räumlich getrennten zweiten Bildbereich, der der Farbe rot zugeordnet ist. Im übrigen weist der Farbteiler einen dichroitischen Spiegel auf, welcher den roten Spektralbereich des auftreffenden Lichts reflektiert und den grünen Spektralbereich durchläßt. Weiterhin ist im Farbteiler ein Rotfilter vorgesehen, das dem dichroitischen Spiegel nachgeordnet ist und im Strahlengang des roten Lichts liegt.

Außerdem gehört zum Farbteiler ein Spiegel, welcher den im grünen Spektralbereich liegenden Teil des auftreffenden Lichts reflektiert. Schließlich ist dem Farbteiler noch ein Grünfilter zugeordnet, das dem Spiegel nachgeordnet ist und im Strahlengang des grünen Lichts liegt.

Von den im Bildprozessor vorgesehenen zwei oder mehr Kanälen ist ein erster Kanal A einem grünen Bildsignalanteil und ein zweiter Kanal B einem roten Bildsignalanteil zugeordnet, und das im ersten Kanal A empfangene Bildsignal wird nach Digitalisierung durch eine Verzögerungseinrichtung um eine erste vorgegebene Verzögerungszeit verzögert, während das im zweiten Kanal B empfangene Bildsignal nach Digitalisierung durch eine zweite Verzögerungseinrichtung um eine zweite vorbestimmte Verzögerungszeit verzögert wird, die sich von der ersten Verzögerungszeit um etwa eine halbe Bildzeile unterscheidet. Auf diese Weise lassen sich die Ausgangssignale von beiden Kanälen A und B, nachdem die darin verarbeiteten Bildsignale mittels eines Digital/Analog-Wandlers in analoge Bildsignale zurückgewandelt worden sind und die jeweils störenden und zeitlich versetzten, der anderen Farbe zugeordneten Bildinformationen durch einen Austastgenerator in beiden Kanälen unterdrückt worden sind, jeweils dem Grün- und Roteingang des RGB-Farbvideomonitors zuführen, der das rot-grüne Bild erzeugt.

In weiteren Ausführungsformen kann Gewebe mit einer speziellen Lichtquelle mit mehr als einer Wellenlänge angeregt werden. Hier ist insbesondere eine zusätzliche Anregung im roten Spektralbereich von Interesse. Das rückgestreute rote Licht liefert ein Reflexionsbild, welches zu einer Qualitätsverbesserung des Fluoreszenzbildes herangezogen werden kann. Es ist auch eine gleichzeitige Anregung der Fluoreszenz mit Anregungslicht in mehreren Spektralbereichen möglich.

Es können Fluoreszenzbilder bei verschiedenen Anregungsspektren gleichzeitig oder hintereinander gewonnen werden. Daraus ist wiederum ein einzelnes Bild, z.B. durch Überlagerung, ableitbar.

Statt der beiden prinzipiell gleichartigen Kanäle des Diagnosegeräts gemäß der bevorzugten Ausführungsform kann das Gerät auch mehr als zwei Verarbeitungskanäle aufweisen, die jeweiligen Farbbildsignalen zugeordnet sind. Außerdem kann das Diagnosegerät zusätzlich den blauen Kanal des RGB-Videomonitors nutzen, z.B. für das oben erwähnte Reflexionsbild.

An direkt zugänglichen Gewebeoberflächen, wie z.B. auf der Haut, äußeren Geschlechtsorganen oder der Mundhöhle, ist das System ebenfalls einsetzbar. In diesem Fall kann auf das Endoskop verzichtet und statt dessen gegebenenfalls ein geeignetes Objektiv verwendet werden.

Die Lichtquelle ist vorzugsweise mit einer Quecksilberdampf-Xenonlampe ausgestattet. Geeignet ist weiterhin eine Xenonlampe. Im übrigen könnte die Lichtquelle auch durch einen Laser bzw. durch mindestens einen Diodenlaser gebildet sein.

Eine Xenonlampe hat zumindest im sichtbaren Bereich ein relativ kontinuierliches Spektrum, weshalb eine solche Lampe zur Weißlichtdarstellung in der Endoskopie besonders geeignet ist. Das Spektrum einer Quecksilberdampf-Xenonlampe setzt - sich im Prinzip zusammen aus dem einer Quecksilberdampflampe und dem einer Xenonlampe. Dabei sind insbesondere die Maxima im blauen/violetten Bereich bei 405 nm und 436 nm für die Anwendung beim erfindungsgemäßen Diagnosegerät von Interesse, da bei diesen Wellenlängen hohe Leistungen zur Fluoreszenzanregung zur Verfügung stehen. Sonstige Leistungsspitzen einer Quecksilberdampf-Xenonlampe, die sich eventuell störend auf das Weißlichtbild auswirken und eine Farbverfälschung des Endoskopbildes ergeben könnten, lassen sich erforderlichenfalls durch zusätzliche Filter reduzieren oder beseitigen.

Nachstehend wird ein Ausführungsbeispiel des erfindungsgemäßen Diagnosegeräts anhand der Zeichnung näher beschrieben. Es zeigen:
- Fig. 1: ein Diagnosegerät in Form schematischer Funktionsblöcke,
- Fig. 2: als Blockschaltbild die beiden Kanäle A und B der Bildverarbeitungsmittel und
- Fig. 3a-c: graphische Bildsignale, wie sie auf dem CCD-Chip in Fig. 1 und in den beiden Kanälen A und B der in Fig. 2 dargestellten Bildverarbeitungsmittel auftreten.

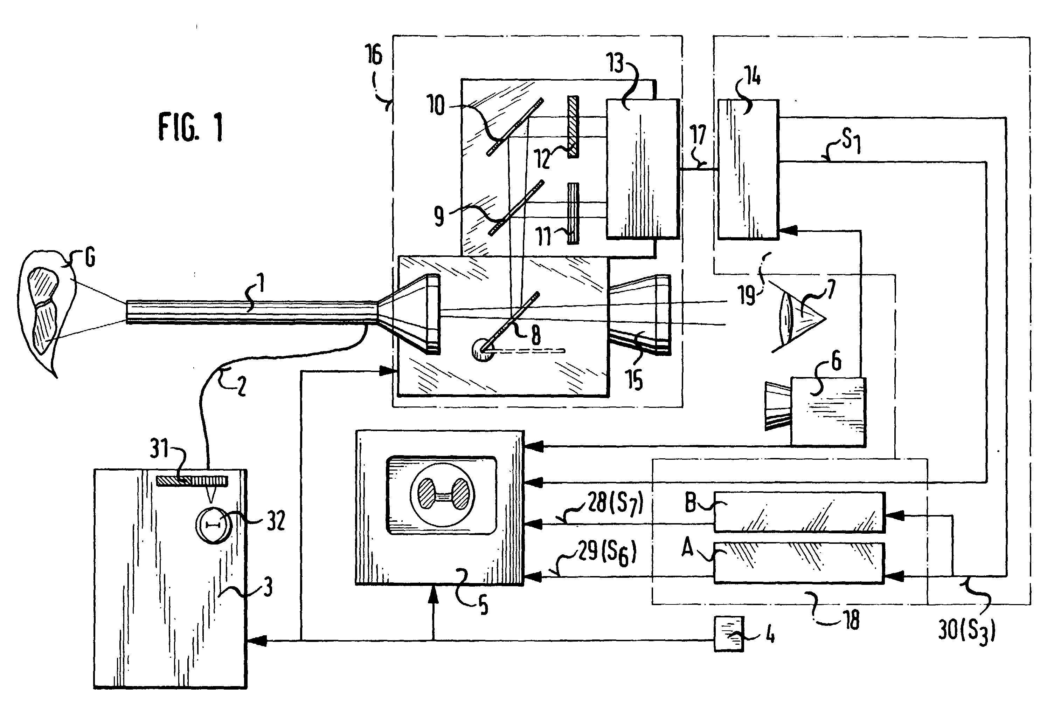

Gemäß Fig. 1 ist in bekannter Weise eine Lichtquelle 3 an, ein Endoskop 1 über einen als Kabel ausgebildeten Lichtleiter 2 angeschlossen. Die Lichtquelle 3 enthält eine Lampe, z.B. eine Quecksilberdampf-Xenonlampe 32, und ein dieser nachgeschaltetes Filter 31, welches ein zur Anregung der Fluoreszenz geeignetes Spektrum durchläßt. Das Filter 31 läßt sich bei Betätigung eines Schalters 4 in und aus dem Strahlengang der Lampe 32 schwenken. Der Schalter 4 befindet sich am Kopfstück 16 des Endoskops 1 oder ist als Fußschalter ausgeführt.

Im ausgeschwenkten Zustand des Filters 31 wird von der Lichtquelle 3 weißes Licht für eine konventionelle Untersuchung eines Gewebebereichs G, z.B. direkt mit dem Auge 7, über das Lichtleitkabel in einen im Endoskop befindlichen Lichtleiter eingestrahlt. Im eingeschwenkten Zustand des Filters 31 liefert die Lichtquelle 3 am distalen Endoskopende austretendes Anregungslicht zur Anregung von Fluoreszenz im Gewebebereich G. Solches fluoreszenzanregendes Licht liegt bevorzugt im ultravioletten und/oder blauen Spektralbereich. Statt eines einzelnen schwenkbaren Filters 31 können auch mehrere Filter hin- und hergeschwenkt werden, wobei ein dafür geeigneter Schalter und Filterantrieb vorgesehen sind.

Auf das Okularteil des Endoskops 1 ist das Kopfstück 16 z.B. mit einem nicht gezeigten Schnappverschluß aufgesetzt. Das Kopfstück 16 enthält einen Klappspiegel 8, der synchron mit dem Filter 31 der Lichtquelle 3 in und aus dem Strahlengang des vom Gewebebereich G reflektierten Lichts schwenkbar ist. Der Klappspiegel 8 ist bei Weißlicht aus dem Strahlengang herausgeschwenkt. Im Fluoreszenzmodus gelangt das von dem Gewebebereich G ausgesandte und vom Endoskop 1 empfangene bildgebende Licht auf einen dichroitischen Spiegel 9, welcher beispielsweise das rote Spektrum reflektiert und einen grünen Spektralanteil durchläßt. Dann gelangt der rote Spektralanteil zunächst auf ein Rotfilter 11, welches die Farbtrennung verbessert, und danach auf eine Hälfte eines CCD-Festkörperchips 13 einer hochempfindlichen Schwarz-Weiß-CCD-Festkörperkamera. Parallel dazu gelangt der durch den dichroitischen Spiegel 9 gegangene grüne Spektralanteil des Lichts über einen Spiegel 10 abgelenkt durch ein Grünfilter 12, welches die Farbtrennung verbessert, auf die andere Hälfte des CCD-Festkörperchips 13. Das Kopfstück 16 enthält weitere nicht gezeigte optische Elemente, z.B. Linsen. Es kann auch ein Zoomobjektiv enthalten, welches es erlaubt, die Bildgröße an unterschiedliche Endoskope oder abzubildende Gewebebereiche anzupassen.

Die Optik im Kopfstück 16 bildet einen Farb-Strahlteiler, der die beiden Rot-Grün-Spektralbereiche des empfangenen Lichts in zwei räumlich getrennte, dem jeweiligen Spektralbereich zugeordnete parallele Bildbereiche auf dem Schwarz-Weiß-CCD-Festkörper 13 der Kamera zerlegt.

Die verwendete Schwarz-Weiß-Kamera ist erheblich empfindlicher als vergleichbare Farbkameras und eignet sich deshalb dazu, auch sehr lichtschwache Fluoreszenzereignisse ohne Bildverstärker aufzunehmen. Sie kann außerdem mehrere Bilder integrieren und damit die Lichtempfindlichkeit weiter steigern.

Das Kopfstück 16 ist über ein Kabel 17 mit einem Elektronikteil 19 verbunden. Dieser besteht aus einer Kameraelektronik 14 und einem Videoprozessor 18. Die Komponenten des Elektronikteils 19 sind z.B. in einem relativ kleinen Tischgehäuse untergebracht.. Der Elektronikteil 19 weist Ausgänge S1, 28, 29 auf, die mit einem standardisierten RGB-Videomonitor 5 verbunden sind. Der Videomonitor 5 liefert ein rotgrünes Bild des mit dem Endoskop 1 beobachteten Gewebebereichs G.

Das Kopfstück 16 weist außerdem ein Okular 15 auf, durch welches bei Weißlicht wahlweise mit dem Auge 7 oder einer endoskopischen Farbkamera 6 konventionell diagnostiziert werden kann.

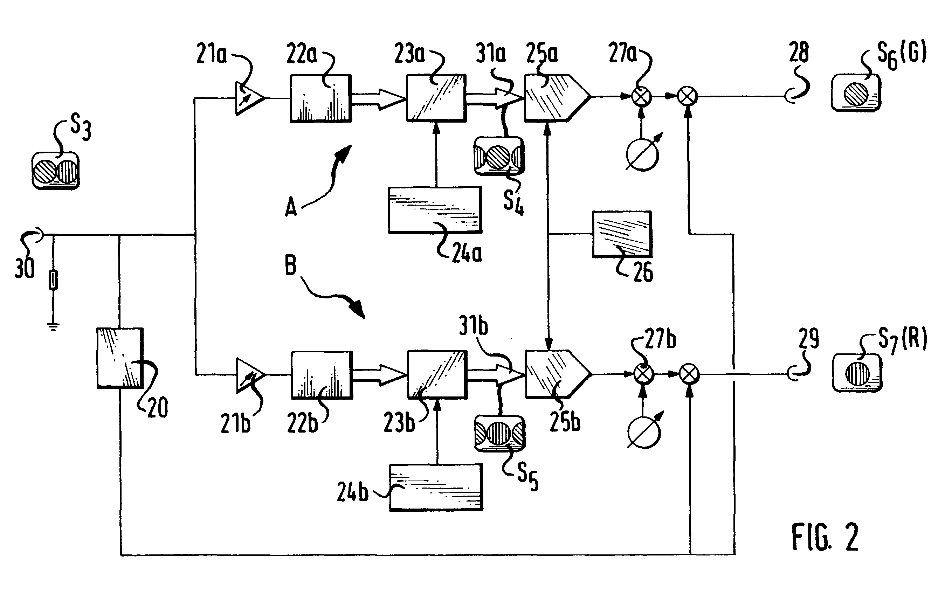

Fig. 2 zeigt den Aufbau des Videoprozessors 18. Er weist einen Standardvideoeingang 30 auf, welcher mit einem Ausgang der Kameraelektronik 14 verbunden ist. Ein Synchronseparator 20 trennt den Synchronisierimpuls S1 des Videosignals ab. Danach wird das Eingangssignal S3 am Eingang 30 in zwei prinzipiell identische Kanäle A und B eingespeist, von denen der Kanal A der Verarbeitung des dem grünen Spektralbereich zugeordneten Abbildungsteils auf dem CCD-Festkörper 13 und der Kanal B der Verarbeitung des dem roten Spektralbereich zugeordneten Abbildungsbereichs auf dem CCD-Festkörper 13 dienen.

In beiden Kanälen A und B wird das Eingangssignal S3 zunächst mit Verstärkern 21a, 21b verstärkt und in Analog-Digital-Umsetzern 22a, 22b in ein digitales Signal umgewandelt. Dieses digitale Signal gelangt dann in jeweils ein First-In-First-Out-(FIFO)-Schieberegister 23a bzw. 23b. Die beiden FIFO-Register 23a, 23b stehen jeweils mit einem Verzögerungsgenerator 24a und 24b in Verbindung. Die Verzögerungszeiten dieser beiden Verzögerungsgeneratoren sind in beiden Kanälen unterschiedlich eingestellt. Die Differenz der Verzögerungszeiten entspricht etwa einer halben Bildzeile. Das digitalisierte Videosignal wird im FIFO-Register 23a des ersten Kanals A um etwa eine Viertelzeile verzögert, während das digitalisierte Videosignal im zweiten FIFO-Register 23b des zweiten Kanals B um etwa eine Dreiviertelzeile verzögert wird. Danach werden die so verzögerten digitalen Videosignale beider Kanäle in Digital/Analogwandlern 25a und 25b wieder in analoge Videosignale zurückgewandelt und jeweils einer Offsetkorrektur durch Korrekturglieder 27a und 27b unterworfen.

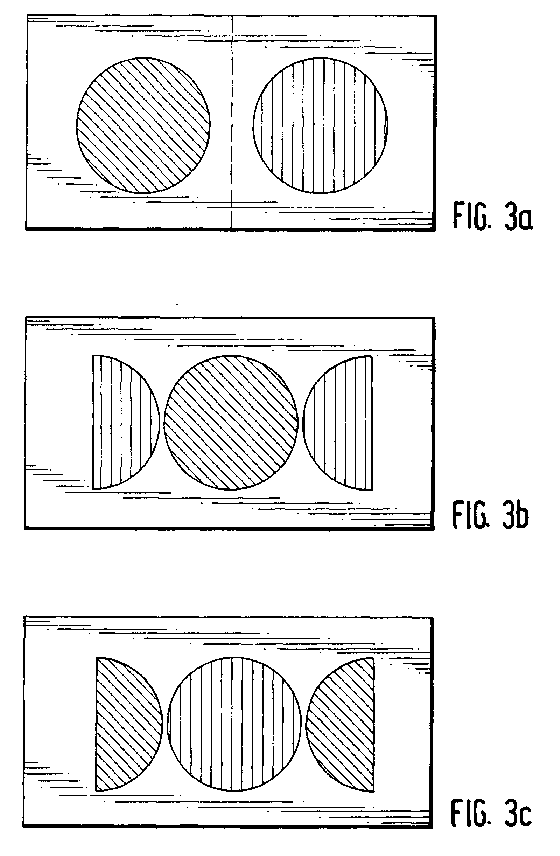

An den Schaltungspunkten 31a und 31b vor den Digital/ Analogwandlern 25a, 25b liegen jeweils digitale Videosignale S4 und S5 vor, die symbolisch dargestellt sind. Beim digitalen Videosignal S4 ist das Bild um eine Viertelzeile nach rechts verschoben, und die grüne Bildinformation befindet sich im Zentrum und ist jeweils von einem halben roten Bildanteil benachbart (vgl. Fig. 3b). Beim digitalen Videosignal S5 ist das Bild nochmals um eine halbe Zeile nach rechts verschoben.

Hier befindet sich die rote Bildinformation im Zentrum, benachbart von den beiden grünen Halbbildern (vgl. Fig. 3c). Fig. 3a zeigt das Bild, wie es auf den CCD-Chip 13 fällt. Ein Austastgenerator 26 unterdrückt in beiden Kanälen A und B jeweils die störende Bildinformation der anderen Farbe. Nach der Offsetkorrektur durch die Offsetkorrekturglieder 27a und 27b wird der Synchronimpuls S1 dem Signal jeweils wieder zugemischt. An den Ausgangsbuchsen 28 und 29 stehen somit jeweils die grüne und die rote Bildinformation S6 und S7 zur Verfügung. Durch Anschluß der Ausgangsbuchsen 28 und 29 an die RGB-Eingänge G "grün" bzw. R "rot" erzeugt der Farbmonitor 5 automatisch ein zweifarbiges Bild. An einen Videoeingang kann auch noch parallel eine konventionelle Farbkamera angeschlossen werden.

An den beiden Ausgängen 28 und 29 des in Fig. 2 dargestellten Bildprozessors 18 erscheinen somit die beiden Bildsignale S6 entsprechend der grünen Bildinformation G und S7 entsprechend der roten Bildinformation R gleichzeitig, so daß sie ohne weiteres an dem Farbvideomonitor 5 überlagert dargestellt werden können.

Das erfindungsgemäße Prinzip kann auch bei einer Anregung eines Gewebebereichs mit mehr als einer Wellenlänge verwendet werden. Insbesondere ist auch an eine zusätzliche Anregung im roten Spektralbereich zu denken. Das vom Gewebebereich zurückgestreute rote Licht liefert ein Reflexionsbild, welches beispielsweise dem blauen Anschluß B des Videofarbmonitors angelegt werden kann.

Ferner ist auch eine gleichzeitige Anregung von Fluoreszenzlicht mit mehreren Spektralbereichen möglich. Es können dabei Fluoreszenzbilder bei verschiedenen Anregungsspektren gleichzeitig oder auch hintereinander gewonnen werden. Daraus kann wiederum ein einzelnes Bild, z.B. durch Überlagerung, abgeleitet werden. Schließlich kann das Gerät auch mit mehr als zwei Kanälen ausgestattet werden.

## Patentansprüche

1. Diagnosegerät zur bildgebenden Aufnahme fluoreszierender biologischer Gewebebereiche (G), insbesondere durch ein Endoskop (1), mit einer Lichtquelle (3, 32), die unter Verwendung einer Filteranordnung (31) wenigstens einen zur Fluoreszenzanregung im Gewebe geeigneten Spektralbereich emittiert, mit einer Optik (8) zur Erfassung und Leitung des vom Gewebe (G) reflektierten Fluoreszenzlichts, mit mindestens einer Videokamera, die Bilder von wenigstens zwei verschiedenen Spektralbereichen des vom Gewebe (G) reflektierten und von der Optik geleiteten Fluoreszenzlichts aufnimmt, und mit Bildverarbeitungsmitteln (18), die durch Verarbeitung der Bildinformation der von der Videokamera aufgenommenen Bilder der verschiedenen Spektralbereiche gemäß einem bestimmten Verarbeitungsalgorithmus in wenigstens zwei getrennten Kanälen (A, B) jeweils zur Diagnose des untersuchten Gewebebereichs dienende farbseparate Bildsignale erzeugen, wobei die farbseparaten Bildsignale an den Ausgängen der getrennten Kanäle (A, B) einem RGB-Farbmonitor (5) eingegeben werden, so daß dieser ein gemischtfarbiges Monitorbild zeigt, und wobei die Videokamera eine hochempfindliche Schwarz-Weiß-CCD-Festkörperkamera ist und einen Farbteiler (9, 10, 11, 12) aufweist, der die mindestens zwei Spektralbereiche des empfangenen Lichts in wenigstens zwei räumlich getrennte, dem jeweiligen Spektralbereich zugeordnete parallele Bildbereiche auf dem Schwarz-Weiß-CCD-Festkörper (13) der Videokamera zerlegt, und wobei die Bildverarbeitungsmittel (18) die räumlich getrennten Bildbereiche auf dem CCD-Festkörper zeitsequentiell empfangen und in den getrennten Kanälen gegeneinander so verzögern, daß an den Ausgängen der Kanäle (A, B) die vorher zeitlich getrennten farbseparaten Bildsignale zeitgleich ausgegeben werden.

2. Diagnosegerät nach Anspruch 1, **dadurch gekennzeichnet, daß** der Farbteiler (9-12) der Festkörperkamera (13) das Fluoreszenzbild des aufgenommenen Gewebebereichs (G) in einen ersten, der Farbe grün zugeordneten Bildbereich und in einen davon räumlich getrennten zweiten Bildbereich zerlegt, der der Farbe rot zugeordnet ist.

3. Diagnosegerät nach einem der Ansprüche 1 oder 2, **dadurch gekennzeichnet, daß** der Farbteiler einen dichroitischen Spiegel (9) aufweist, welcher den roten Spektralbereich des auftreffenden Lichts reflektiert und den grünen Spektralbereich durchläßt.

4. Diagnosegerät nach Anspruch 3, **dadurch gekennzeichnet, daß** der Farbteiler weiterhin ein Rotfilter (11) aufweist, das dem dichroitischen Spiegel (9) nachgeordnet ist und im Strahlengang des roten Lichts liegt.

5. Diagnosegerät nach einem der Ansprüche 1 bis 4, **dadurch gekennzeichnet, daß** der Farbteiler einen Spiegel (10) aufweist, welcher den im grünen Spektralbereich liegenden Teil des auftreffenden Lichts reflektiert.

6. Diagnosegerät nach Anspruch 5, **dadurch gekennzeichnet, daß** der Farbteiler ein Grünfilter aufweist, das dem Spiegel (10) nachgeordnet ist und im Strahlengang des grünen Lichts liegt.

7. Diagnosegerät nach einem der Ansprüche 1 bis 6, **dadurch gekennzeichnet, daß** die Bildverarbeitungsmittel (18) zwei prinzipiell gleichartige Kanäle (A, B) aufweisen, von denen der erste Kanal (A) einem grünen Bildsignalanteil und der zweite Kanal (B) einem roten Bildsignalanteil zugeordnet sind, und daß das im ersten Kanal (A) empfangene Bildsignal digitalisiert und durch eine erste Verzögerungseinrichtung (23a, 24a) um eine erste vorgegebene Verzögerungszeit verzögert wird und das im zweiten Kanal empfangene Bildsignal digitalisiert und durch eine zweite Verzögerungseinrichtung (23b, 24b) um eine zweite vorbestimmte Verzögerungszeit verzögert wird, die sich von der ersten Verzögerungszeit um etwa eine halbe Bildzeile unterscheidet.

8. Diagnosegerät nach Anspruch 7, **dadurch gekennzeichnet, daß** die verarbeiteten digitalen Bildsignale beider Kanäle (A, B) mittels eines Digital/Analog-Wandlers (25a, 25b) in analoge Bildsignale gewandelt werden, und daß ein Austast-Generator (26) vorgesehen ist, der in jedem der beiden Kanäle (A,B) die störende, zeitlich versetzte Bildinformation, die der anderen Farbe zugeordnet ist, unterdrückt.

9. Diagnosegerät nach Anspruch 7 oder 8, **dadurch gekennzeichnet, daß** die beiden farbseparaten Bildsignale (S6, S7) jeweils dem Grün- bzw. Roteingang (G, R) des RGB-Farbmonitors (5) eingegeben werden.

10. Diagnosegerät nach einem der Ansprüche 1 bis 9, **dadurch gekennzeichnet, daß** die Lichtquelle (3) eine Quecksilberdampf-Xenonlampe (32) aufweist.

11. Diagnosegerät nach einem der Ansprüche 1 bis 9, **dadurch gekennzeichnet, daß** die Lichtquelle (3) eine Xenonlampe (32) aufweist.

12. Diagnosegerät nach einem der Ansprüche 1 bis 9, **dadurch gekennzeichnet, daß** die Lichtquelle (3) durch einen Laser gebildet ist.

13. Diagnosegerät nach einem der Ansprüche 1 bis 9, **dadurch gekennzeichnet, daß** die Lichtquelle durch mindestens einen Diodenlaser gebildet ist.

## Claims

1. Diagnostic apparatus for taking pictures of fluorescent biological tissue regions (G), in particular through an endoscope (1), with a light source (3, 32) which with the use of a filter arrangement (31) emits at least one spectral range capable of exciting fluorescence in the tissue, with an optical system (8) for picking up and guiding the fluorescent light reflected by the tissue (G), with at least one video camera which takes pictures of at least two different spectral ranges of the fluorescent light reflected by the tissue (G) and guided by the optical system, and with image processing means (18) which, by processing the image data of the pictures taken by the video camera in the different spectral ranges in accordance with a defined processing algorithm in at least two separate channels (A, B) each used for the diagnosis of the tissue region examined, generate colour-separated picture signals, the colour-separated picture signals at the outputs of the separate channels (A, B) being fed to an RGB colour monitor (5) so that this monitor displays a mixed-colour picture, and the video camera being a highly sensitive black-and-white CCD solid-state camera and having a colour splitter (9, 10, 1 1, 12) which separates the at least two spectral ranges of the light received into at least two spatially separated, parallel picture regions, each assigned to its respective spectral range, on the black-and-white CCD solid[-state device] (13) of the video camera, and in that [sic] the image processing means (18) receive the spatially separated picture regions on the CCD solid[-state device] sequentially in time and retard them with respect to each other in the separate channels so that the colour- separated picture signals which have hitherto been separated in time are output isochronously at the outputs of the channels (A, B).

2. Diagnostic apparatus according to Claim 1, **characterized in that** the colour splitter (9-12) of the solid-state camera (13) separates the fluorescent image of the target tissue region (G) into a first picture region assigned to the colour green and a second, spatially separate, picture region assigned to the colour red.

3. Diagnostic apparatus according to Claim 1 or Claim 2, **characterized in that** the colour splitter has a dichroic mirror (9) which reflects the red spectral range of the incident light and transmits the green spectral range.

4. Diagnostic apparatus according to Claim 3, **characterized in that** the colour splitter also has a red filter (11) subordinated to the dichroic mirror (9) and located in the ray path of the red light.

5. Diagnostic apparatus according to any one of Claims 1 to 4, **characterized in that** the colour splitter has a mirror (10) which reflects the part of the incident light which lies in the green spectral range.

6. Diagnostic apparatus according to Claim 5, **characterized in that** the colour splitter has a green filter subordinated to the mirror (10) and located in the ray path of the green light.

7. Diagnostic apparatus according to any one of Claims 1 to 6, **characterized in that** the image processing means (18) have two fundamentally similar channels (A, B) of which the first channel (A) is assigned to a green picture signal component and the second channel (B) is assigned to a red picture signal component, and **in that** the picture signal received in the first channel (A) is digitized and retarded by a first delay device (23a, 24a) by a first specified delay time and the picture signal received in the second channel is digitized and retarded by a second delay device (23b, 24b) by a second predetermined delay time which differs from the first delay time by approximately half a scanning line.

8. Diagnostic apparatus according to Claim 7, **characterized in that** the processed digital picture signals of both channels (A, B) are converted into analogue picture signals by means of a digital/analogue converter (25a, 25b), and **in that** a blanking generator (26) is provided which in each of the two channels (A, B) suppresses the unwanted time-displaced image data assigned to the other colour.

9. Diagnostic apparatus according to Claim 7 or Claim 8, **characterized in that** the two colour-separated picture signals (S6, S7) are fed to the green and red inputs (G, R), respectively, of the RGB colour monitor (5).

10. Diagnostic apparatus according to any one of Claims 1 to 9, **characterized in that** the light source (3) has a mercury vapour xenon lamp (32).

11. Diagnostic apparatus according to any one of Claims 1 to 9, **characterized in that** the light source (3) has a xenon lamp (32).

12. Diagnostic apparatus according to any one of Claims 1 to 9, **characterized in that** the light source (3) is formed by a laser.

13. Diagnostic apparatus according to any one of Claims 1 to 9, **characterized in that** the light source (3) is formed by at least one diode loser.

## Revendications

1. Appareil de diagnostic destiné à enregistrer, en en donnant des images, des zones de tissu biologique fluorescentes (G), notamment au travers d'un endoscope (1), comportant une source de lumière (3, 32) qui, moyennant l'utilisation d'un dispositif de filtrage (31), émet au moins une plage spectrale propre à stimuler une fluorescence dans le tissu, une optique (8) destinée à capter et conduire la lumière de fluorescence réfléchie par le tissu (G), au moins une caméra vidéo, qui enregistre des images d'au moins deux plages spectrales distinctes de la lumière de fluorescence réfléchie par le tissu (G) et conduite par l'optique, et des moyens de traitement d'images (18) qui, par traitement de l'information des images, enregistrées par la caméra vidéo, des plages spectrales distinctes, conformément à un algorithme de traitement déterminé, produisent, dans au moins deux canaux séparés (A, B), des signaux image différenciés en couleur, qui servent chacun au diagnostic de la zone de tissu examinée, les signaux image différenciés en couleur étant alors, aux sorties des canaux séparés (A, B), introduits dans un présenteur vidéo couleur en RVB (5), afin que celui-ci affiche une image de contrôle à couleurs mêlées, et la caméra vidéo étant une caméra à semi-conducteurs à CCD noir et blanc, de haute sensibilité, et comprenant un séparateur de couleurs (9, 10, 11, 12) qui fractionne les deux plages spectrales, au moins, de la lumière reçue en au moins deux zones d'image parallèles, séparées spatialement et associées à la plage spectrale respective, sur l'élément à semi-conducteurs à CCD noir et blanc (13) de la caméra vidéo, et en ce que les moyens de traitement d'images (18) reçoivent, suivant une séquence temporelle, les zones d'image séparées spatialement sur l'élément à semi-conducteurs à CCD et les retardent suffisamment l'une par rapport à l'autre, dans les canaux séparés, pour qu'aux sorties des canaux (A, B), les signaux image différenciés en couleur, précédemment séparés temporellement, soient délivrés en même temps.

2. Appareil de diagnostic selon la revendication 1, **caractérisé en ce que** le séparateur de couleurs (9 à 12) de la caméra à semi-conducteurs (13) fractionne l'image, obtenue par fluorescence, de la zone de tissu enregistrée (G), en une première zone d'image, associée à la couleur verte, et en une seconde zone d'image séparée spatialement de la première, qui est associée à la couleur rouge.

3. Appareil de diagnostic selon l'une des revendications 1 ou 2, **caractérisé en ce que** le séparateur de couleurs comprend un miroir dichroïque (9), qui réfléchit la plage spectrale rouge de la lumière incidente et laisse passer la plage spectrale verte.

4. Appareil de diagnostic selon la revendication 3, **caractérisé en ce que** le séparateur de couleurs comprend, par ailleurs, un filtre rouge (11), qui est disposé en aval du miroir dichroïque (9) et se place dans le trajet de rayonnement de la lumière rouge.

5. Appareil de diagnostic selon l'une des revendications 1 à 4, **caractérisé en ce que** le séparateur de couleurs comprend un miroir (10) qui réfléchit la partie, située dans la plage spectrale verte, de la lumière incidente.

6. Appareil de diagnostic selon la revendication 5, **caractérisé en ce que** le séparateur de couleurs comprend un filtre vert, qui est disposé en aval du miroir (10) et se place dans le trajet de rayonnement de la lumière verte.

7. Appareil de diagnostic selon l'une des revendications 1 à 6, **caractérisé en ce que** les moyens de traitement d'images (18) comportent deux canaux (A, B) semblables par principe, dont le premier (A) est associé à une fraction verte du signal image et le second (B) à une fraction rouge du signal image, et **en ce que** le signal image reçu dans le premier canal (A) est numérisé et est retardé, par un premier retardateur (23a, 24a), d'un premier temps de retard pré-établi et le signal image reçu dans le second canal est numérisé et est retardé, par un second retardateur (23b, 24b), d'un second temps de retard prédéterminé, qui se différencie du premier temps de retard d'une demi-ligne de balayage de l'image, environ.

8. Appareil de diagnostic selon la revendication 7, **caractérisé en ce que** les signaux image numériques, traités, des deux canaux (A, B), sont convertis en signaux image analogiques au moyen d'un convertisseur numérique/analogique (25a, 25b), et **en ce qu'**il est prévu un générateur d'effacement (26) qui, dans chacun des deux canaux (A, B) supprime l'information d'image parasite, décalée dans le temps, qui est associée à l'autre couleur.

9. Appareil de diagnostic selon la revendication 7 ou 8, **caractérisé en ce que** les deux signaux image (S6, S7), différenciés en couleur, sont introduits respectivement dans l'entrée pour le vert et l'entrée pour le rouge (V, R) du présenteur vidéo couleur en RVB (5).

10. Appareil de diagnostic selon l'une des revendications 1 à 9, **caractérisé en ce que** la source de lumière (3) comprend une lampe au xénon à vapeur de mercure (32).

11. Appareil de diagnostic selon l'une des revendications 1 à 9, **caractérisé en ce que** la source de lumière (3) comprend une lampe au xénon (32).

12. Appareil de diagnostic selon l'une des revendications 1 à 9, **caractérisé en ce que** la source de lumière (3) est formée par un laser.

13. Appareil de diagnostic selon l'une des revendications 1 à 9, **caractérisé en ce que** la source de lumière est formée par au moins un laser à diode.
